# EUROPEAN PATENT SPECIFICATION

(11) **EP 1 145 178 B1**
(45) Date of publication and mention of the grant of the patent: **22.03.2006**
(21) Application number: 00914442.9
(22) Date of filing: 25.01.2000
(51) Int. Cl.: G06F 19/00

(54) **METHOD FOR SELECTING OLIGONUCLEOTIDES HAVING LOW CROSS-HYBRIDIZATION**
VERFAHREN ZUR AUSWAHL VON OLIGONUKLEOTIDEN MIT NIEDRIGER KREUZHYBRIDISIERUNG
TECHNIQUE PERMETTANT DE SELECTIONNER DES OLIGONUCLEOTIDES A FAIBLE HYBRIDATION CROISEE

(30) Priority: 25.01.1999 US 116956 P
(43) Date of publication of application: 17.10.2001
(73) Proprietor: Combimatrix Corporation, Burlingame, CA 94010 (US)
(72) Inventor: ANDERSON, Brooke, P., Redmond, WA 98052 (US); MONTGOMERY, Donald, D., Millbrae, CA 94030 (US)
(74) Representative: Schüssler, Andrea
(86) International application number: PCT/US2000/002000
(87) International publication number: WO 2000/043942

(56) References cited:
- EP-A- 0 799 897
- US-A- 5 403 707
- US-A- 5 716 784

## Description

### FIELD OF THE INVENTION

The present invention is in the field of biological and chemical synthesis and processing. The present invention relates to methods for selecting oligonucleotides for low cross hybridization. The present invention may be applied in the field of, but is not limited to, the field of chemical or biological synthesis, diagnostics and therapeutics.

The present application claims priority to U.S. Provisional Patent Application Serial No. 60/116,956 filed January 25, 1999.

### BACKGROUND OF THE INVENTION

Advances are emerging continually in the field of biological and chemical processing and synthesis. Many novel and improved solid phase arrays or "gene chips" are being developed providing rapid methods for synthesizing chemical and biological materials. Examples of such technologies include those described by Pirrung *et al.*, U.S. Patent No. 5,143,854, those described by Southern in WO 93/22480, those described by Heller in WO 95/12808 and those described by Montgomery in PCT/US97/11463. Hence, it is possible to synthesize, to manipulate and to examine ever increasing amounts of genetic materials. Moreover, it is possible to work simultaneously with, to analyze, and to test ever larger amounts of genetic materials.

Oligonucleotides can hybridize or bind to other oligonucleotides depending upon whether or not their sequences are more or less complementary. Sometimes, it is desirable to find a set of oligonucleotides that, as much as possible within a given set of constraints, do not hybridize or bind to each other. Optimization methods can be used to assist in selecting such a set of oligonucleotides.

There are many possibilities of how to make an oligonucleotide or oligonucleotides from a longer oligonucleotide. For example, one may cut the long oligonucleotide or select a segment or segments from which to form a smaller oligonucleotide. These smaller oligonucleotides formed by these and other methods known to those skilled in the art may be referred to as "substring sequences". There is great flexibility on which substring of the oligonucleotide to select as a target sequence for binding.

Mitsuhashi *et al*., U.S. Patent No. 5,556,749 describe a computerized method for designing optimal DNA probes. The method is intended to produce probes designed for diagnosis and monitoring. However, the method described therein does not contemplate choosing more than one probe for simultaneous use with another probe whereby interaction and cross hybridization is minimized.

EP 0 799 897 A1 pertains to a method of selecting nucleic acids tags and corresponding probes for microarrays. The tags are used to label and detect compositions including cells and viruses. The components are labeled with such selected nucleic acid tags and the presence of individual tags is monitored by hybridization to a probe array. Thus, the tag nucleic acids are labels for the individual components. The tag nucleic acids are synthesized. Thus, the tag nucleic acids can be freely selected out of all possible combinations of bases. Tags are selected by eliminating tags which bind to the same target with a similar energy of hybridization. Tags bind complementary nucleic acids with a similar energy of hybridization when a complementary nucleic acid to one tag binds to another tag with an energy exceeding a specified threshold value. Thereby cross-hybridization should be prevented. These tags are used for labeling cells and viruses. They are even used for labeling DNA-sequences, *e*.*g*. "deletion mutants". This known method provides a set of tags and a set of probes which are complementary to each other and which are designed in such a way that cross-hybridization should be avoided.

US 5,403,707 discloses the use of an oligonucleotide as "a probe" which is substantially complementary to a nucleic acid sequence of a target nucleic acid and which is used for detection or capture of an amplified target nucleic acid. Where a multiplicity of probes are used simultaneously for capturing a multiplicity of amplified target nucleic acids all of the capture probes have Tₘ's which differ by no more than about 15°C. Preferably the capture probe Tₘ's used simultaneously differ by no more than about 5°C.

US 5,716,784 teaches a process for replication and homogeneous assay detection of a target nucleic acid sequence in a sample, wherein an oligonucleotide analytical probe and an oligonucleotide detection probe are used which differ in nucleotide length so that the Tₘ's are at least 10°C apart. This method employs a two-component fluorescence reporter with one component on each probe type resulting in an alteration of fluorescence when the analytical probe hybridizes with the target nucleic acid. The difference in length of the two probes ensures that in the presence of a target the two probes separate, the longer analytical probe hybridizing with the target.

It is an object of the present invention to provide a method for choosing complementary substrings from among target oligonucleotides such that the substrings bind relatively well to their target oligonucleotides but do not substantially bind to other oligonucleotides in a sample. For instance, given a long oligonucleotide (*e*.*g*., a string of RNA, mRNA, DNA or cDNA) it is possible to select a piece or pieces from it for a later experiment, *e*.*g*. as a capture probe. In the case of an immobilized capture probe, it is desirable that the oligonucleotide substring sequence bind to the long oligonucleotide that it came from but not to other oligonucleotides that might be present in a sample. It is often desirable to prepare an array of such immobilized capture probes so that each one binds or hybridizes to its intended target but does not bind or hybridize strongly to any of the other targets in the solution. An array of immobilized capture probes constructed according to the methods described allows using a smaller array of capture-probes for sequestering a desired set of targets because the array does not require as much redundancy for elucidating data points as other arrays where binding is not as clearly discernible.

### SUMMARY OF THE INVENTION

In a first aspect, the present invention features methods to provide a set of probes that hybridize or bind relatively well to their intended targets but do not bind substantially to unintended targets. The quality of hybridizing or binding relatively well to intended targets but not to unintended targets is quantified using delta Tm (difference in melting temperatures) according to the methods of the present invention. A probe having a relatively small delta Tm generally hybridizes to at least one unintended target substantially well. A probe having a relatively large delta Tm has substantially no unintended targets that it hybridizes to substantially well.

The method according to the present invention which is defined in the claims features:
1. Determining a set of targets. In some circumstances where it is not clear what the identity of all the targets in a particular solution might be, it is possible to determine a list of some of the targets that might be in the particular solution and to include that list in the set of targets. If there are some targets in the list that are not actually in the solution, it does not harm the quality of probes selected according to the present methods.
2. Selecting a particular target from the set of targets. This becomes the current target
3. Choosing a sequence substring from the current target and providing its complementary sequence. This is a candidate probe. Choosing a sequence substring may be done by starting at a particular point in the current target and then incrementing the starting point by some amount each time a new substring is chosen, with wraparound of the starting point to the front portion of the current target when an increment would otherwise run off the end of the target. A substring may be chosen at random or any arbitrary function may be applied in order to determine which substring to choose. If there are no more substrings in the current target that have not already been tried, it is recommended to: (i) use the best candidate probe selected so far noting that this target might not be as selectively captured as desired, (ii) to do no more picking of probes for this target, and (iii) to return to step 2, *supra.* Otherwise, using the candidate probe as determined and proceeding to step 4, *infra.*
4. Determining whether the candidate probe satisfies any criteria required or desired for the set of probes. If it does not meet such criteria, returning to step 3 and choose a new candidate probe. If it does satisfy such criteria, proceeding to step 5.
5. Calculating the Tm for the candidate probe using the hybridization model. Calculating substantially all possible cross Tm's of the candidate probe hybridizing to all unintended targets and finding the maximum cross Tm. Calculating delta Tm. Note that the set of all unintended targets will include previously picked probes if probes are also to be in solution as opposed to affixed to a support.
6. Proceeding to step 7 if delta Tm is acceptably large. Returning to step 3 and choosing a new candidate probe if delta Tm is not acceptable large.
7. At this point, a suitable probe has been chosen. The foregoing procedure may be repeated additional times to choose other probes for additional targets or, if desired, additional probes for a target for which one has already found one or more probes.

In a second aspect, the present invention features a programmed computer system for providing the sequences of a set of probes that hybridize or bind relatively well to their intended targets but do not bind substantially to unintended targets.

### DETAILED DESCRIPTION OF THE INVENTION

The present invention features methods to provide a set of probes that hybridize or bind relatively well to their intended targets but do not bind substantially to unintended targets. The quality of hybridizing or binding relatively well to intended targets but not to unintended targets is quantified using delta Tm according to the methods of the present invention. A probe with a small delta Tm generally hybridizes to at least one unintended target relatively well. A probe having a large delta Tm has substantially no unintended targets that it hybridizes to relatively well.

The methods according to the present invention feature:
1. Determining a set of targets. In some circumstances where it is not clear what the identity of all the targets in a particular solution might be, it is possible to determine a list of some of the targets that might be in the particular solution and to include that list in the set of targets. If there are some targets in the list that are not actually in the solution, it does not harm the quality of probes selected according to the present methods.
2. Selecting a particular target from the set of targets. This becomes the current target for which one desires a probe.
3. Choosing a sequence substring from the current target and providing its complementary sequence. This is a candidate probe. Choosing a sequence substring may be done by starting at a particular point in the current target and then incrementing the starting point by some amount each time a new substring is chosen, with wraparound of the starting point to the front portion of the current target when an increment would otherwise run off the end of the target. A substring may be chosen at random or any arbitrary function may be applied in order to determine which substring to choose. If there are no more substrings in the current target that have not already been tried, it is recommended to: (i) use the best candidate probe selected so far noting that this target might not be as selectively captured as desired, (ii) to do no more picking of probes for this target, and (iii) to return to step 2, *supra.* Otherwise, using the candidate probe as determined and proceeding to step 4, *infra.*
4. Determining whether the candidate probe satisfies any criteria required or desired for the set of probes. If it does not meet such criteria, returning to step 3 and choose a new candidate probe. If it does satisfy such criteria, proceeding to step 5.
5. Calculating the Tm for the candidate probe using the hybridization model. Calculating substantially all possible cross Tm's of the candidate probe hybridizing to all unintended targets and finding the maximum cross Tm. Calculating delta Tm. Note that the set of all unintended targets will include previously picked probes if probes are also to be in solution as opposed to affixed to a support.
6. Proceeding to step 7 if delta Tm is acceptably large. Returning to step 3 and choosing a new candidate probe if delta Tm is not acceptable large.
7. At this point, a suitable probe has been chosen. The foregoing procedure may be repeated additional times to choose other probes for additional targets or, if desired, additional probes for a target for which one has already found one or more probes.

In particular embodiments where the probes and targets are both in solution (*i.e.,* the probes are not tethered to a support), it is preferable to include each previously accepted probe into the list of targets before calculating delta Tm for the current candidate probe. Those skilled in the art will readily understand that it is preferable to do so because the probes are able to hybridize to each other as well as to the targets and so should be included in any calculation of the strength of unintended hybridizations. Those skilled in the art will readily understand that this feature means that the present invention provides probes having reduced cross hybridization with each other. Therefore, the present invention is particularly applicable to instances where multiple probes are used simultaneously in solution. This is often the case where primers are being used such as to amplify or copy sections of genetic material. In that case, "probe" and "primer" are meant to refer to the same oligonucleotide.

Adding targets as the methods according to the present invention progress may interfere with the ability to find a probe for a particular target not because of conflict with other targets but because of conflict (too strong a hybridization) to previously picked probes. If performing the methods outlined in the present invention reveals that there are a relatively large number of unacceptable probes found, and if these probes are unsuitable because they bind too strongly to other probes, it is generally preferred to begin the method from the start using a different order of picking current targets. This generally results in a different order of picking probes and can result in a set of probes that are more mutually exclusive. For instance, if the first time, probes for targets are picked in the following order: target 1, target 5, target 2, target 4, target 3, and the best probes for target 2 and target 3 do not have an acceptable delta Tm because they bind relatively well to probes for target 1 and target 5, it is possible to repeat the methods according to the present invention again by picking probes for targets in for example, the following order: target 2, target 3, target 5, target 4, target 1. In effect, the sequence for choosing targets may be modified within the present methods. Alternatively, it is possible to start with the set of probes found so far, look at the probe that was found to be less than ideal, find what other probes it hybridizes too strongly with, redo those probes, and repeat this process until a compatible set of probes is found. By way of example, suppose that a set of probes is found, but probes 2 and 5 do not have an acceptable delta Tm because they hybridize too strongly with probes 1 and 3, respectively. It is possible to eliminate probe 1 and proceed according to the methods of the present invention again for target 1 leaving the rest of the probes as is, finding a new acceptable probe for target 1 that perhaps does not conflict with probe 2. Then a skilled artisan may do the same for probe 3. This process (of redoing the previously found probes that later are found to conflict with other probes) may be iterated until a good solution is found. If no suitable probes are found, one or more targets may be eliminated from the solution of interest.

The method of the present invention provides a set of probes that hybridize or bind relatively well to their intended targets but do not bind substantially to unintended targets. The quality of hybridizing or binding relatively well to intended targets but not to unintended targets may be quantified using delta Tm. A probe having a small delta Tm generally hybridizes to at least one unintended target relatively well. A probe having a large delta Tm has substantially no unintended targets that it hybridizes to relatively well. Therefore, the set of probes according to the present invention may have, for example, a delta Tm of 5° C, 10° C or, for greater separation, 20° C. In general, the larger the delta Tm, the easier to dehybridize unintended targets while maintaining the intended targets hybridized by the probes in the subject set of probes.

In a second aspect, the present invention features a programmed computer system for providing the sequences of a set of probes that hybridize or bind relatively well to their intended targets but do not bind substantially to unintended targets. Such a programmed computer system may comprise any one of a large number of possible software programs that may be designed by those skilled in the art without undue experimentation. Such a programmed computer system comprises a means for determining or designating one or more particular targets from a set of targets to probe (a current target), a means for determining or designating a sequence substring from the current target and determining its complementary sequence (a candidate probe). The means for choosing a sequence substring may function by starting at a particular point in the current target and then incrementing the starting point by some amount each time a new substring is chosen. A substring may be chosen at random or any arbitrary function may be applied in order to choose which substring to pick by the computer means, a means for determining whether the candidate probe satisfies any criteria required or desired for probes, and a means for calculating the Tm for the candidate probe using a hybridization model.

As used herein, the following terms are understood to mean the following:

A "target" is an oligonucleotide in a sample.

A "probe" is an oligonucleotide intended to bind or hybridize to a target. Note that in cases where probes and targets are in solution, a particular oligonucleotide can be both a probe and a target. A "set of probes" is intended to include two or more probes. Preferably, a "set of probes" includes 10 or more probes. More preferably, a "set of probes" includes 100 ormore probes. Even more preferably, a "set of probes" includes 1000 or more probes.

The "intended target" of a probe is the target that it is designed to best hybridize to. Generally, this will be the target from which the probe is a complementary substring. For example, if GATTACAGATTACA is a particular oligonucleotide in solution (or target), one possible substring is CAGAT. The complement of the substring CAGAT is ATCTG, and thus ATCTG can be a probe for hybridizing to the intended target GATTACAGATTACA.

An "unintended target" is a target other than the intended target.

"Cross hybridization" is hybridization of a probe to a target other than its intended target or to another probe.

"Tm" is most preferably the melting temperature of hybridization of a probe to its intended target. Melting temperature is defined in scientific literature and is used herein to describe a measure of how strongly a probe hybridizes to a target.

"Cross Tm" is the melting temperature of hybridization of a probe to an unintended target (or to another probe). For melting-temperature models that are location dependent, it is preferable to use the location where the melting temperature of hybridization is highest.

"Constraints" are the conditions or qualifications that must be substantially met when choosing probes. In most instances, "constraints" refer to a feature or property of the probe. For example, it might be desirable to select only those probes having a Tm between about 50 ° and 60 ° C or that do not contain more than three G's in a row. There may be any number of heuristics or constraints imposed by preferences on the practitioner to use the probe set. Some exemplary constraints include that all probes are a particular length (*e.g.*, 20 bases long or 30 bases long) or that all probes have Tm's within a particular range (*e.g*., within 5° C of each other or within 2° C of a mean Tm for probes having a particular length). In the case of using probes as primers, there can typically be constraints that the probe must bind to the target sequence within a certain area (in order to do the priming task correctly). Other constraints might be that the probe is not to have G's and C's within the last four base positions of its 3' end, and so on.

"delta Tm" is the difference, for a particular probe, between Tm and the maximum cross Tm.

A "hybridization model" is a mathematical model by which one calculates an estimated Tm or cross Tm based on the probe, the oligonucleotide to which it hybridizes and possibly the position of the hybridization. The hybridization model may also require the input of solution concentrations or additional factors. An important feature of a "hybridization model" is that it provides an estimate of Tm or cross Tm algorithmically. There are many hybridization models discussed in scientific literature and are believed applicable within the scope of the methods of the present invention. Likewise, additional custom hybridization models may be created.

"Acceptable delta Tm" is the smallest delta Tm that is determined to be acceptable for a probe to be accepted according to the methods of the present invention. For example, an acceptable delta Tm might be 5° C, 10° C or, for greater separation, 20° C. Likewise, an acceptable delta Tm might be chosen as any number in between. In general, the larger the delta Tm, the easier to separate unacceptable from acceptable probes. Similarly, the larger the delta Tm, the easier to dehybridize unintended targets while maintaining the intended targets hybridized.

As used herein the term "bind relatively well to intended targets" is understood to describe a feature whereby a probe does not separate from but rather remains hybridized to a target sequence under normal operating conditions. A preferred example of such a feature is a perfectly complementary probe that does not separate from but rather remains hybridized to a target sequence at temperatures under 80° C

As used herein the term "does not bind substantially to unintended targets" is understood to describe a feature whereby a probe does not hybridize to target sequences other than those to which it possesses a high degree of complementarity under normal operating conditions. A preferred example of a such a feature is a perfectly complementary probe that does not separate from but rather remains hybridized to a target sequence at temperatures below 80° C. However, the same probe does not bind to or easily separates from a target sequence to which it does not possess a high degree of complementarity at temperatures greater than some temperature significantly below 80° C, such as greater than 70° C, greater than 65° C, greater than 60° C, greater than 50 C, etc. The feature is intended to include minimal hybridization that may be reversed by agitation or heating above room temperature.

### DESCRIPTION OF THE PREFERRED EMBODIMENTS

The following are provided purely by way of example and are not intended to limit the scope of the present invention.

### EXAMPLE 1

### Selecting oligonucleotides for low cross hybridization

The following is a sample list of targets for designing probes. The probes were to be built on a DNA chip such as those used in accordance with the method described by Montgomery in WO 98/01221, attached to a layer on the surface of a chip some other substrate so that the probes themselves are not floating around in solution. Thus, we do not account for probes in the target set during operation of the method.
>gi|6717738|gb|AW305385.1|AW305385 xv93h12.x1 NCI_CGAP_Brn53 Homo sapiens cDNA clone IMAGE:2826119 3', mRNA sequence 
GCCAGTCACATGCTTACCTGCATTTTTAAAGACAGCTTTCAGGTATTTGGGGACTACATTA
TTACCAAAC
CTTGGCTTTGGGAGATTATACAGGTCCGAGGAACTCGTGTCTACTGCAGACGAATGCAAT
TACCCCACCT
TCCTCCATACAGAATTGTTAGGAAATGTCCACTCCTTTGGGGGTGATTTTTCTCCTCAAGT
TGTAGCCAA
CATTTTGTCCGTAACTGATTTCAGGGCAAACATTTCTGACATCTTCCTCCAGCTCAGTCTG
CCATGCCTT
GGCAATCCAGTTTCCTGTCATATGCGAGCCATCCAAGTTGATGCCAAGTAAGATTTGCCC
AGCTCAAAGT
GAAAGTGTTTGCGTCTTGGTATCCGGAATCCTCAGCCCCAGTAGCAAAGCTTTAGTCATTC
ACCTTCATC
 >gi|6717590|gb|AW305237.1|AW305237 xr79h11.x1 NCI_CGAP_Lu26 Homo sapiens cDNA clone IMAGE:2766405 3', mRNA sequence 
ACTACTATACGGCTGCGAGAAGACGACAGAAGGGTCATGTGTTAACTATAATCACATTTA
TGGTTTGGAA
CCATCACCCCAAGGTAAAAAAAAAATAAAAGGTATTCCCAGGTATGTTTGGCAAAATAA
AATAAAGGTAA
TTAAAAACCGAAAAAAAAAAAAAAAAAAAAAAAAAAAAAAGTCGTATCGATGT
 >gi|6714107|gb|AW3Q4418.1|AW304418 xv60f12.x1 NCI_CGAP_Lu28 Homo sapiens cDNA clone IMAGE:2817551 3' mRNA sequence 
CTTTTTCCTTTTATTCACTCCCAGCAGATCTTTCTTTTTCCTGTAAGCTTACCACTTCTAAA
TTTAATAT
GTGTTTTGAGCTCATTATTTAAAGGAATCACATCTTGCTAATCACATCCAAGGCACCGGA
ACATAGTGTC
TATACTGACTGAACAGGCCAAGCTTCGTGAGTTAATTAATAAAATATTTGGTAAGAAACG
GTCCATCATT
ATCTTATCACTTGAGATGACAATGTTGAAACTTACAGGATGGAAGGCATCTCATTAATTC
AGACCATTTC
AAATCAATTTTATTTTGACTTACAGTCTTGAAATAACATATCATTATCTTTGGCCATATCA
AAAACTGAA
CCCAGTTGGAAAATATTTATATGTCCAAATATTGGTTTAGAGGAAAGTATAGCATGTTTTT
GGTAAAT
 >gi|6713707|gb|AW304018.1|AW304018 xv15h11.x1 Soares_NFL_T_GBC_S1 Homo sapiens cDNA clone IMAGE:2813253 3', mRNA sequence 
TTTTTTACAGGATAATACTTTAATTACGAAAAGCACAAATTATGTATCATTGGAACCAGC
AAACACACAG
TAGCAGGAAGGATGCTTGCTCCCAAGGCTCTTCAGTCATCAGAGGACACACTCAAGCCCC
ACCTGAGTCT
TCTCCCCATTCCATCGGCCATCCCTGCTCAGGATGTGGTACCAGGGCCATTCCCAACAGCC
TCATCTCAG
TAGACTCCAGTTTGTCTAATTCTCCTTCAATGGTGCTCCTTCGTCACTTCTCGTGGGCTGGC
GGATAG
 >gi|6713507|gb|AW303818.1|AW303818 xr23d05.x1 NCI_CGAP_Ut4 Homo sapiens cDNA clone IMAGE:2760969 3', mRNA sequence 
GAACTTTGAATGTGCTTTATTATGCCACAAATTCCCAGGAGATTTAAGAAATAGTATTTCT
GAACAGGAA
TAATAATTTCACAAATACTAACACTTTATTGACAATAGACAAGTCTTTTAGGGTAGTGCA
CATGTACTTA
AAAACTACCTTCTACCAATCTCAACACTTTTTATAAATTTTCAGGTGAAACTGTAGCAGAT
CCTACTTTA
TTTTTCAATGGTTAGTGTAAAATTCTGTATGTAAAATAAGTACATATTTTGAGATGGAAGA
AGGACTGCA
TGTGAAATGCTTTGCCTAAGTTGTAAGGCTCCTGTCTTTACGCTATCATTAAAGGCCAAAA
AAATCACTG
CTAGAAATGTTCCCCAAAAAATTCTTAAACAGCTCAGTCTTTAAAAGTATTAATAATTTTT
TTTTTTTTT
TTTTTTGGAGACAGAGTTTCGCTCTTCTTGCCCAGGCTGGAGTACAATGGCGCAATCTCAG
CTCACCG
 >gi|6712898|gb|AW303218.1|AW303218 xr59g03.x1 NCI_CGAP_Ov26 Homo sapiens cDNA clone IMAGE:2764468 3' similar to contains Alu repetitive element;, mRNA sequence 
TATACGGCTGCGATAAGACGACAGAAGGGGTAGGACTGAGGCCTGAGTACACCTTTTAT
ATTTTGGACAT
TTACGTATTAAAAAAATTATCTAGCTGGGCATGGTGGCACACACCTATGGTCCCAGCTGC
TTGGGAGGCT
GAAGTAGGAGGCTGGCTTGAGCCCAGGAGTTTAAGTCCAGCCAGAGCAACATAGTGAGA
ATTCATCTCAA
GAAAAAAAAAAGAAAAAAAAAAAAAAGAAAAAAGTCGTATCGA
 >gi|6711895|gb|AW302218.1|AW302218 xs03d05.x1 NCI_CGAP_Kid11 Homo sapiens cDNA clone IMAGE:2768553 3' similar to TR:Q14934 Q14934 NF-AT3. ;, mRNA sequence 
CATATTACTGGTCATTGAGCAGTTTATTGGGAGCAATCTGACCCCAGGTTGCCAGCACAA
CAGCCAGCCC
ACACTCTAGACACGCCTTCACTCCAGTCCATTCTGGCACCTAGCCTCAGTCTTCACCCTCC
TCCCTCCTC
CACACACTCCTTCCCCCAGCCCTCCAAGGCAGCACCAGGCCTGAGGGCCACACCTCAGCT
GGGGGAGGGG
AGGGAAGACAGTGAGACAGACAGAAGCTGGGGAAAGAGGAGCCAGGGTTGGCCCCAAG
CTTCTGTAGCCA
CCACTCCAGGAAGGAGGGAAAGGGGGCAGGGCTGAGGCTGGGGCTGGGGTTGCCAGGTG
ATGACAGTTCA
 
CGTGGTTCAGGCAGGAGGCTCTTCTCCAGGAGGTGCAGGGAAGCCACTCAGGTCTCGGCC
AATGATCTCA
CTCACTGTAAAGGAGGGGCAGTTGAGAGACTGGGCTA
 >gi|6710695|gb|AW301018.1|AW301018 xk11e01.x1 NCI_CGAP_Co20 Homo sapiens cDNA clone IMAGE:2666424 3', mRNA sequence 
GCCCTTTTTTTTTTTTTCAACAAGGAAATGTATTTATTTTTCTTTAGAATTTGGCTCAG
AACAGTAA
CAAAAATATTTACATTAAAATAAATTAACATGCAATTACTTAACCATATGTAATAATTTA
CGTTGGAATA
TATTAGCCTTCCCATGAGTTTAATAAAAACTAATATTTGGTTTTAGATTCAATACCATCCT
TTCAAATAT
TTGGGTATGAAACTTGGTAGCAATGCAATTGTCTGATGTACAGAGCAGATTTCACCATGA
GAGATTACAC
CAAAGAACAGATGTCCCTTCCCAGAACATTATCTCACCCCAGACTCAGAAACTGAGCAGC
CAAGCTTCCT
TCCCAGGAATCACCATGGAATGTCTGAACAATAACCAGGCCCTGGAGATTACTGCAGGGC
TGGCAGAGTT
TTAGGAATCAGCCAAACTC
 >gi|6710495|gb|AW300818.1|AW300818 xk06e09.x1 NCI_CGAP_Co19 Homo sapiens cDNA clone IMAGE:2665960 3' similar to TR:088814 088814 HEME-BINDING PROTEIN. ;, mRNA sequence 
AAGTCAATGCCTTTTATTTTTAGTTTTTCTGAAGACAAAGCTCTTATAAGAATCACAGATG
AAAGATCAG
GCACAAATCACATTTTCCCCCTTAATAACAAAATACAAATCCAATAATTTTAGAAAATCA
GTTTTTAGTG
ACCCAGATGCCTGGAGAAAAGCTGCCAGGATTTTTCTGGTCTATCGCAGAATTTTCTACA
TCAATGAGAA
GGATGCTGCATATCTTGGCTGTATTATTTCCTACCGTGAGAAAAGAGACTTAGTATATGG
AACATGCTTT
TTTCAGAAAATTGGCAGTAACTGACTTTGAAGGAAAGTTGGTTAAGTTGGACTTGCAGCT
GGAACTTGGG
AAGCACTGTCCCCTCCTTACCCCCGAGGAAGGAGACACAGAGGCACACTTCCAGTAAGTT
CTTGGTTCAG
TGGGTCACTCATGTCTTCAACAGCCAGATCTCATTGCGCCGTCCGTAGGGCTTCATGGGA
GGGTCATAAC
CCGTGCAGAAATAGATGTCCCNCCGGTAGGGTGCTGTGCCCTTCAAGGGAGCACGCAA
 >gi|6705458|gb|AW298822.1|AW298822 UI-H-BW0-ajq-h-09-0-UI.s1 NCI_CGAP_Sub6 Homo sapiens cDNA clone IMAGE:2732800 3', mRNA sequence

CGGCCGCGCCGGTTTTTTTCAAGTTTTGGGTATGTTTAATCTGTTATGTACTACTGTTCTG
TTTGTTAT
TGTTTTGTTAATTACACCATAATGCTAATTTAAAGAGACTCCAAATCTCAATGAAGCCAG
CTCACAGTGC
TGTGTGCCCCGGTCACCTAGCAAGCTGCCGAACCAAAAGAATTTGCACCCCGCTGCGGGC
CCACTTGGTT
GGGGCCCTGCCCTGGCAGGGTCATCCTGTGCTCGGAGGCCATCTCGGGCATAGGTCCACC
CCGCCCCACC
CCTCCAGAACACGGCTCACGCTTACCTCAACCATCCTGTTTGCGGCGTCTGTCTGAACCAC
GCGGGGGCC

TTGAGGGACGCTTTGTCTGTCGTGATGGGGCAAGGGCACAAGTCCTGAATGTTGTGTGTA
TCGAGAGGCC
AAAGGCTGGTGGCAA

We used the following constraints. Probes must be 20 bases long. Probes must have a Tm within 1°C of the expected Tm for a 20-mer according to the hybridization model used (in this case 68.25° C).

We used the following hyridization model: Tm = 81.5 + 0.41 * Pgc - 675 / N - Pmm, where Pgc is the percent GC content of the probe = (number of G's + number of C's) / N * 100, N is the length of the probe in bases, and Pmm is the percent mismatches = (number of mismatches) / N * 100.

We chose an acceptable delta Tm of 20° C.

The algorithm worked as follows. We began with target 1. We picked a 20-mer out of it at a randomly selected location and found its complement as a candidate probe. We checked that the candidate probe satisfied the constraints. If not, we chose another 20-mer from a random location. If it did, we then calculated the Tm's for this probe hybridizing to all other targets at all other locations and used that data to find the maximum cross Tm and thus delta Tm. If delta Tm was greater than or equal to 20° C, we kept this probe and obtained a probe for the next target (target 2). If not, we chose another 20-mer from a random location. We repeated this process until we found one acceptable probe for each target.

The following is the list of probes found by this process. In the following, there is header information given for each probe indicating from which target it comes (i.e., what its intended target is), where in that target the probe comes from (i.e., at what offset into the intended target), the Tm of the probe, the maximum cross Tm of the probe, what unintended target provides the maximum cross Tm, and where in that unintended target the maximum cross Tm happens (at what offset).

Note that the Tm values given all match exactly. The experimentally determined Tm's will not necessarily match exactly -- the Tm's given are estimated Tm's derived from the hybridization model, which in this case results in the methods described being able to find probes that all match exactly in estimated Tm.
> probe 1 from target 1 at offset 359; Tm = 68.3; max. cross Tm = 33.3 from target 9 at offset 253 ATTCCGGATACCAAGACGCA > probe 2 from target 2 at offset 2; Tm = 68.3; max. cross Tm = 48.3 from target 6 at offset -3 CTTCTCGCAGCCGTATAGTA > probe 3 from target 3 at offset 145; Tm = 68.3; max. cross Tm = 28.3 from target 5 at offset 272 AAGCTTGGCCTGTTCAGTCA > probe 4 from target 4 at offset 239; Tm = 68.3; max. cross Tm = 23.3 from target 1 at offset 176 AAGTGACGAAGGAGCACCAT > probe 5 from target 5 at offset 424; Tm = 68.3; max. cross Tm = 28.3 from target 1 at offset 25 GAGCGAAACTCTGTCTCCAA > probe 6 from target 6 at offset 36; Tm = 68.3; max. cross Tm = 28.3 from target 1 at offset 225 AAAGGTGTACTCAGGCCTCA > probe 7 from target 7 at offset 333; Tm = 68.3; max. cross Tm = 28.3 from target 1 at offset 314 ACGTGAACTGTCATCACCTG > probe 8 from target 8 at offset 352; Tm = 68.3; max. cross Tm = 28.3 from target 5 at offset 24 CATTCCATGGTGATTCCTGG > probe 9 from target 9 at offset 210; Tm = 68.3; max. cross Tm = 33.3 from target 4 at offset 79 AGCCAAGATATGCAGCATCC > probe 10 from target 10 at offset 350; Tm = 68.3; max. cross Tm = 28.3 from target 1 at offset 175

ACAGACAAAGCGTCCCTCAA

### EXAMPLE 2

In this example, the list of targets was the same as in Example 1. Likewise, all of the parameters and the model used for calculating Tm were the same as in Example 1. The only difference was that we used a variation of the method.

We began with target 1. We picked a 20-mer out of it at a randomly selected location and found its complement as a candidate probe. We determined that the candidate probe satisfied the constraints. If not, we picked another 20-mer from the "next" location, *supra.* If it did, we calculated the Tm's for this probe hybridizing to all other targets at all other locations and used that data to find the maximum cross Tm and thus delta Tm. If delta Tm was greater than or equal to 20° C, we kept this probe and moved on to getting a probe for the next target (target 2). If delta Tm was not greater than or equal to 20° C, we went back and picked another 20-mer from the "next" location (see below). We repeated this process until we found one acceptable probe for each target.

By "next location," we applied the following process. We selected a new candidate probe starting at a location one base to the right (in the 3' direction) of the previous pick. If such a location resulted in not having enough bases to make a candidate probe (such as when the next location is too close to the end of the target so that there are not enough bases left to make a probe of the desired length), we started at location 1 of the target. Thus, the process of scanning a target for an acceptable probe was started at a randomly selected point and then progressed incrementally along the target with wrap-around to the front of the target when the end was reached.

This process provides an exhaustive search of a target for an acceptable probe. It will find an acceptable probe if one exists. Thus, it is a good candidate search method for situations where the targets might be very similar except for small differences (perhaps mutations) at particular sites in the oligonucleotide.
This process resulted in the following set of probes being found.
> probe 1 from target 1 at offset 62; Tm = 68.3; max. cross Tm = 33.3 from target 3 at offset 372 CCCAAAGCCAAGGTTTGGTA > probe 2 from target 2 at offset 0; Tm = 68.3; max. cross Tm = 38.3 from target 6 at offset -5 TCTCGCAGCCGTATAGTAGT > probe 3 from target 3 at offset 115; Tm = 683; max. cross Tm = 33.3 from target 6 at offset 173 TATGTTCCGGTGCCTTGGAT > probe 4 from target 4 at offset 234; Tm = 683; max. cross Tm = 333 from target 1 at offset 399 ACGAAGGAGCACCATTGAAG > probe 5 from target 5 at offset 292; Tm = 68.3; max. cross Tm = 33.3 from target 7 at offset 111 GGAGCCTTACAACTTAGGCA > probe 6 from target 6 at offset 154; Tm = 68.3; max. cross Tm = 33.3 from target 3 at offset 73 TTAAACTCCTGGGCTCAAGC > probe 7 from target 7 at offset 265; Tm = 68.3; max. cross Tm = 28.3 from target 4 at offset 93 AGTGGTGGCTACAGAAGCTT > probe 8 from target 8 at offset 379; Tm = 68.3; max. cross Tm = 28.3 from target 1 at offset 59 ATCTCCAGGGCCTGGTTATT > probe 9 from target 9 at offset 438; Tm = 68.3; max. cross Tm = 38.3 from target 4 at offset 194 CGCAATGAGATCTGGCTGTT > probe 10 from target 10 at offset 350; Tm = 68.3; max. cross Tm = 28.3 from target 1 at offset 175

ACAGACAAAGCGTCCCTCAA

## Claims

1. A method to provide a set of probes for an array for sequestering a desired set of targets wherein the probes hybridize to their intended targets but do not substantially hybridize to unintended targets in a sample mixture of intended targets and unintended targets, comprising the steps of:
(a) determining a set of targets;
(b) determining a particular current target from the set of targets to probe;
(c) choosing a sequence substring from the current target and providing its complementary sequence, which becomes the candidate probe;
(d) determining that a candidate probe satisfies any criteria desired or required for probes;
(e) calculating the Tm for the candidate probe using a hybridization model;
(f) calculating substantially all possible cross Tm's of the candidate probe hybridizing to all unintended targets and finding the maximum cross Tm;
(g) calculating delta Tm;
(h) determining whether the delta Tm is at least. 5 °C; and
(i) repeating steps (b) forward until the desired probes are found.

2. The method of claim 1 wherein choosing a sequence substring is performed by starting at a particular point in the current target and then shifting the starting point by one base in the 3'-direction each time a new substring is chosen.

3. The method of claim 1 wherein the substring is chosen at random.

4. The method of claim 1 wherein the delta Tm is at least 20 °C.

5. The method of claim 1 wherein the delta Tm is at least 10 °C.

6. The method according to one of the preceding claims, wherein the targets are DNA-sequences or RNA-sequences and the probes are oligonucleotides.

7. The method according to one of the claims 1 to 6, wherein the set of probes includes 100 or more probes.

8. The method according to one of the claims 1 to 6, wherein the set of probes includes 1000 or more probes.

9. The method according to one of the preceding claims, wherein each found probe is included in the set of targets defined in step (a) and considered by repeating steps (b) forward.

10. The method according to one of the preceding claims, wherein the probes are used as immobilized capture probes.

11. A programmed computer system for providing the sequences of a set of probes that hybridize to their intended targets but do not substantially hybridize to unintended targets in a sample mixture of intended targets and unintended targets, wherein the programmed computer system performs the method according to one of the claims 1 to 9.

## Patentansprüche

1. Verfahren zur Bereitstellung eines Probensatzes für einen Array zum Herausziehen eines gewünschten Targetsatzes, wobei die Proben in einer Prüfgutmischung aus spezifischen und unspezifischen Targets mit ihren spezifischen Targets hybridisieren, aber mit unspezifischen Targets im Wesentlichen nicht hybridisieren, welches Verfahren die folgenden Schritte umfasst:
(a) Bestimmen eines Targetsatzes;
(b) Bestimmen eines bestimmten, aktuellen Targets aus dem zu untersuchenden Targetsatz;
(c) Auswählen einer Sequenzteilkette aus dem aktuellen Target und Bereitstellen ihrer Komplementärsequenz, die zu der Kandidatenprobe bzw. Probe engerer Wahl wird;
(d) Bestimmen, dass eine Kandidatenprobe alle Kriterien erfüllt, die für Proben erwünscht oder erforderlich sind;
(e) Berechnen der Tm für die Kandidatenprobe unter Verwendung eines Hybridisierungsmodells;
(f) Berechnen von im Wesentlichen allen möglichen Misch-Tm's der Kandidatenprobe, die mit allen unspezifischen Targets hybridisiert, und Ermitteln der maximalen Misch-Tm;
(g) Berechnen von Delta Tm;
(h) Bestimmen, ob Delta Tm mindestens 5 °C beträgt; und
(i) Wiederholen der Schritte bis (b), bis die gewünschten Proben ermittelt sind.

2. Verfahren nach Anspruch 1, wobei das Auswählen einer Sequenzteilkette vonstatten geht, indem an einem bestimmten Punkt im aktuellen Target gestartet wird und dann der Startpunkt jedes Mal, wenn eine neue Teilkette gewählt wird, um eine Base in der 3'-Richtung verschoben wird.

3. Verfahren nach Anspruch 1, wobei die Teilkette zufällig ausgewählt wird.

4. Verfahren nach Anspruch 1, wobei die Delta Tm mindestens 20 °C beträgt.

5. Verfahren nach Anspruch 1, wobei die Delta Tm mindestens 10 °C beträgt.

6. Verfahren nach einem der vorhergehenden Ansprüche, wobei die Targets DNA- oder RNA-Sequenzen und die Proben Oligonukleotide sind.

7. Verfahren nach einem der vorhergehenden Ansprüche 1 bis 6, wobei der Probensatz 100 oder mehr Proben umfasst.

8. Verfahren nach einem der vorhergehenden Ansprüche 1 bis 6, wobei der Probensatz 1000 oder mehr Proben umfasst.

9. Verfahren nach einem der vorhergehenden Ansprüche, wobei jede ermittelte Probe in den in Schritt (a) definierten Probensatz aufgenommen wird und durch Wiederholung der Schritte bis (b) berücksichtigt wird.

10. Verfahren nach einem der vorhergehenden Ansprüche, wobei die Proben als immobilisierte Einfangproben verwendet werden.

11. Programmiertes Computersystem zur Bereitstellung der Sequenzen eines Satzes aus Proben, die in einer Prüfgutmischung aus spezifischen und unspezifischen Targets mit ihren spezifischen Targets hybridisieren, aber mit unspezifischen Targets im Wesentlichen nicht hybridisieren, wobei das programmierte Computersystem das Verfahren nach einem der Ansprüche 1 bis 9 ausführt.

## Revendications

1. Une méthode pour fournir une série de sondes au cours d'une étude pour isoler une série choisie de cibles dans laquelle les sondes s'hybrident aux cibles désignées et ne s'hybrident pas essentiellement aux cibles non désignées dans un mélange d'échantillons pour des cibles désignées et non désignées comprenant les étapes suivantes:
a) déterminer une série de cibles,
b) déterminer une cible courante particulière à partir d'une série de cibles à sonder,
c) choisir une sous-chaîne de séquence à partir de la cible courante et fournir sa séquence complémentaire, laquelle devient la sonde candidate,
d) déterminer qu'une sonde candidate satisfait à tous les critères désirés ou requis pour les sondes,
e) calculer le Tm pour la sonde candidate en utilisant un modèle d'hybridation,
f) calculer essentiellement tous les Tm croisés possibles de la sonde candidate s'hybridant à toutes les cibles non désignées et trouver le Tm croisé transversal maximum,
g) calculer le delta Tm,
h) déterminer si le delta Tm est au moins de 5 °C, et
i) répéter encore l'étape b) jusqu'à ce que la sonde désirée soit trouvée.

2. Méthode selon la revendication 1, dans laquelle le choix d'une sous-chaîne de séquence est réalisé en partant au point particulier dans la cible courante et puis on déplace le point de départ à l'aide d'une base dans la direction 3' à chaque fois qu'une nouvelle sous-chaîne est choisie.

3. Méthode selon la revendication 1, dans laquelle la sous-chaîne est choisie au hasard.

4. Méthode selon la revendication 1, dans laquelle le delta Tm est au moins de 20 °C.

5. Méthode selon la revendication 1, dans laquelle le delta Tm est au moins de 10 °C.

6. Méthode selon l'une des revendications précédentes, dans laquelle les cibles sont des séquences ADN ou des séquences ARN et les sondes des oligonucléotides.

7. Méthode selon l'une des revendications 1 à 6, dans laquelle la série de sondes comporte 100 ou plus.

8. Méthode selon l'une des revendications 1 à 6, dans laquelle la série de sondes comporte 1000 ou plus.

9. Méthode selon l'une des revendications précédentes, dans laquelle chaque sonde trouvée est intégrée dans une série de cibles dans l'étape (a) et prise en considération en répétant les étapes (b) suivantes.

10. Méthode selon l'une des revendications précédentes, dans laquelle les sondes sont utilisées comme sondes de capture immobilisées.

11. Système de programme informatique pour fournir les séquences d'une série de sondes qui hydrolysent leurs cibles affectées mais qui n'hydrolysent pas sensiblement les cibles non affectées dans un mélange d'échantillons de cibles désignées et de cibles non désignées, dans lequel le système de programme informatique met en oeuvre la méthode selon les revendications 1 à 9.
